# EUROPEAN PATENT SPECIFICATION

(11) **EP 3 361 967 B1**
(45) Date of publication and mention of the grant of the patent: **03.09.2025**
(21) Application number: 16782169.3
(22) Date of filing: 03.10.2016
(51) Int. Cl.: A61B 17/30

(54) **INSTRUMENT HANDLE AND REPLACEABLE TIP**
INSTRUMENTENGRIFF UND AUSWECHSELBARE SPITZE
POIGNÉE D'INSTRUMENT ET EMBOUT REMPLAÇABLE

(30) Priority: 12.10.2015 US 201562240220 P
(43) Date of publication of application: 22.08.2018
(73) Proprietor: Katalyst Surgical, LLC, Chesterfield, MO 63005 (US)
(72) Inventor: SCHELLER, Gregg D., Wildwood, MO 63038 (US); BASS, Eric J., Webster Groves, MO 63119 (US)
(74) Representative: Carl Zeiss AG - Patentabteilung
(86) International application number: PCT/US2016/055150
(87) International publication number: WO 2017/066026

(56) References cited:
- WO-A1-98/37819
- WO-A2-02/41796
- US-A1- 2013 071 507
- US-A1- 2014 066 977

## Description

### CROSS-REFERENCE TO RELATED APPLICATIONS

This Application claims the benefit of U.S. Provisional Application No. 62/240,220, filed October 12, 2015.

### FIELD OF THE INVENTION

The present disclosure relates to a medical device, and, more particularly, to a surgical instrument.

### BACKGROUND OF THE INVENTION

A variety of surgical procedures are performed through a very small surgical incision in a particular tissue. Reducing the size of a surgical incision during a surgical procedure generally reduces the amount of trauma to the surgical site and generally facilitates faster wound healing. In order to perform surgical procedures through a very small surgical incision, a surgeon may require specialized surgical instruments configured to fit through the very small surgical incision and provide the surgeon with a surgical utility. Sometimes a surgeon may require a surgical utility that may not be easily controlled close to a particular surgical site, e.g., closing forceps jaws inside of an eye. It is generally desirable for a surgeon to be able to control such a surgical utility with a minimal amount of effort. For example, if a surgical utility is controlled by a lever or a switch on an instrument handle, a surgeon may need to adjust an orientation of a surgical instrument in order to actuate the lever or the switch. Additionally, if a surgical utility control mechanism requires a surgeon to apply a significant amount of force to a portion of a surgical instrument, then it may be difficult for the surgeon to manipulate the surgical utility control mechanism without unintentionally moving a portion of the surgical instrument. Ophthalmic surgical instruments are generally categorized as either reusable or single-use. A single-use instrument is typically sterilized prior to sale and is shipped to a surgery center sterile and ready for use in a surgical procedure. A reusable instrument is typically shipped to a surgery center non-sterile and is sterilized by the surgery center between uses in surgery. Reusable instruments are generally lower in overall cost for a surgery center compared to single-use instruments. Single-use instruments offer a surgery center greater convenience compared to reusable instruments. Accordingly, there is a need for an instrument that offers a surgery center the convenience of a single-use instrument at the overall lower cost of a reusable instrument.

US 2013/071507 A1 discloses a steerable laser probe including a handle having a handle distal end and a handle proximal end, a housing sleeve disposed in an inner bore of the handle configured to project a distance from the handle distal end, an optic fiber disposed in the housing sleeve, a shape memory sleeve disposed over a distal end of the optic fiber, and a light source configured to connect to a proximal end of the optic fiber.

US 2014/066977 A1 discloses a micro surgical handle and instrument including an actuation structure having an actuation structure distal end and an actuation structure proximal end, a plurality of actuation arms of the actuation structure, and an actuation structure base.

WO 02/41796 A2 discloses a composite instrument comprising a first functional instrument and a second functional instrument when the first functional instrument is coupled with the second functional instrument.

WO 98/37819 A1 discloses a single use ultrasonic surgical device including a transmission component adapted to receive ultrasonic vibration from a transducer assembly and to transmit the ultrasonic vibration from a first end to a second end.

### BRIEF SUMMARY OF THE INVENTION

The present invention relates to an instrument with the features of claim 1. Advantageous embodiments of the invention are disclosed by the features of the dependent claims.

The present disclosure provides an instrument handle and replaceable tip. In one or more embodiments, an instrument handle and replaceable tip may comprise a reusable instrument handle and a single-use instrument tip. Illustratively, the instrument tip may comprise an outer base, a nosecone, a pressure mechanism, a hypodermic tube, a blank, and a fixation mechanism. In one or more embodiments, the instrument handle may comprise an actuation structure, a fixation mechanism receptacle, and an instrument tip housing. Illustratively, the fixation mechanism and the fixation mechanism receptacle may be configured to temporarily fix the instrument tip in the instrument tip housing. In one or more embodiments, a compression of the actuation structure may be configured to actuate the hypodermic tube relative to the blank. Illustratively, the instrument tip may be removed from the instrument tip housing after use by removing the fixation mechanism from the fixation mechanism receptacle.

### BRIEF DESCRIPTION OF THE DRAWINGS

The above and further advantages of the present invention may be better understood by referring to the following description in conjunction with the accompanying drawings in which like reference numerals indicate identical or functionally similar elements:
FIGs. 1A, 1B, and 1C are schematic diagrams illustrating a transitory element;
FIG. 2 is a schematic diagram illustrating an exploded view of an instrument tip assembly;
FIGs. 3A and 3B are schematic diagrams illustrating an assembled instrument tip;
FIG. 4 is a schematic diagram illustrating an exploded view of an instrument handle assembly;
FIGs. 5A and 5B are schematic diagrams illustrating an assembled instrument handle;
FIG. 6 is a schematic diagram illustrating an exploded view of an instrument assembly;
FIGs. 7A and 7B are schematic diagrams illustrating an instrument with open jaws;
FIGs. 8A and 8B are schematic diagrams illustrating an instrument with partially closed jaws;
FIGs. 9A and 9B are schematic diagrams illustrating an instrument with closed jaws.

### DETAILED DESCRIPTION OF AN ILLUSTRATIVE

### EMBODIMENT

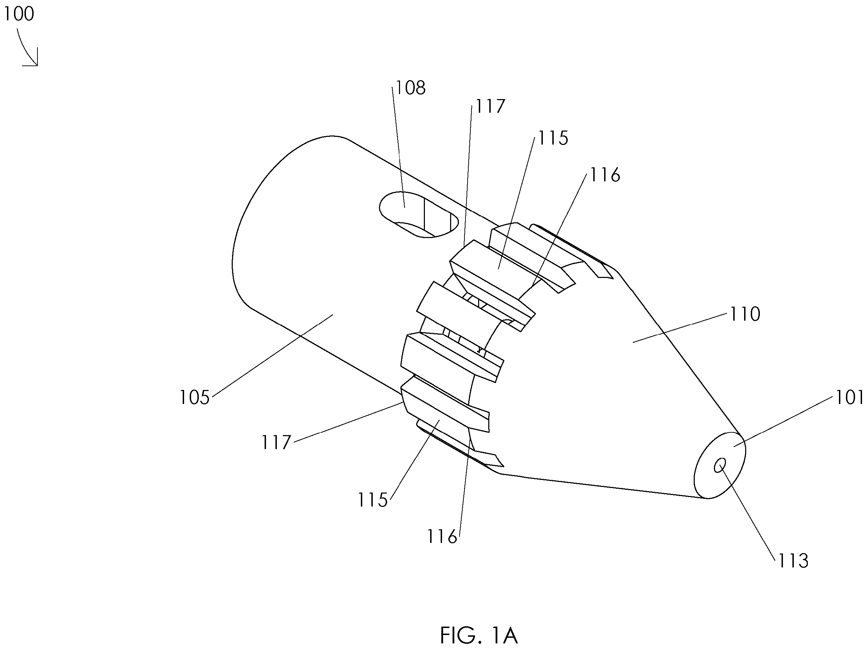

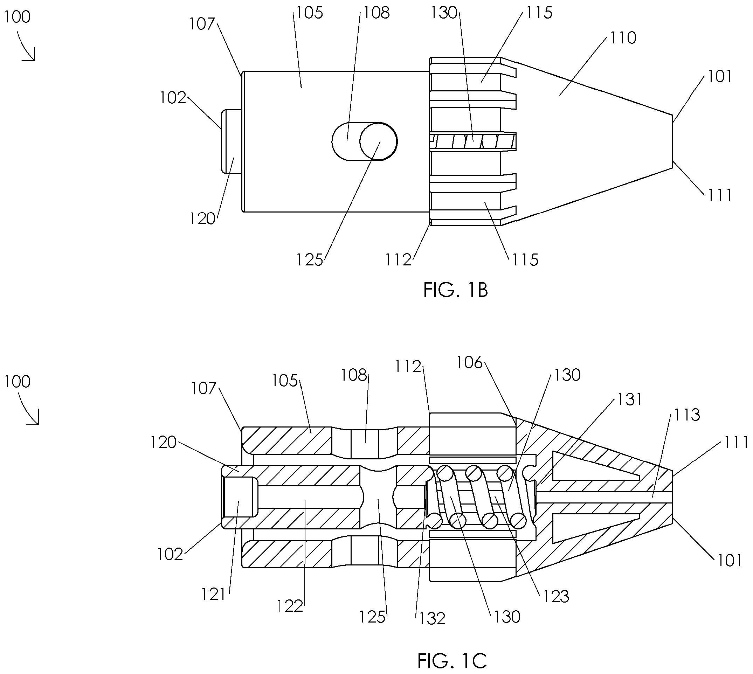

The following conversions apply in the description: Centigrade = (Fahrenheit - 32)/1.8; 1 inch = 25.4 mm; 1 kg = 2.2 pounds. FIGs. 1A, 1B, and 1C are schematic diagrams illustrating a transitory element 100. FIG. 1A illustrates an isometric view of a transitory element 100. FIG. 1B illustrates a superior view of a transitory element 100. FIG. 1C illustrates a cross-sectional view in a sagittal plane of a transitory element 100. Illustratively, a transitory element 100 may comprise a transitory element distal end 101 and a transitory element proximal end 102. In one or more embodiments, transitory element 100 may comprise an outer base 105, a nosecone 110, and a fixation mechanism distal receptacle 120. Illustratively, outer base 105 may comprise an outer base distal end 106 and an outer base proximal end 107. In one or more embodiments, outer base 105 may comprise an outer base aperture 108. Illustratively, nosecone 110 may comprise a nosecone distal end 111 and a nosecone proximal end 112. In one or more embodiments, nosecone 110 may comprise a hypodermic tube housing 113 and a plurality of radial projections 115. Illustratively, each radial projection 115 of the plurality of radial projections 115 may comprise a radial projection distal end 116 and a radial projection proximal end 117. In one or more embodiments, each radial projection 115 of the plurality of radial projections 115 may be separated from at least one radial projection 115 of the plurality of radial projections 115 by an aperture. Illustratively, nosecone 110 may comprise a plurality of apertures. In one or more embodiments, a portion of outer base 105 may be disposed in a portion of nosecone 110, e.g., outer base distal end 106 may be disposed in nosecone 110. Illustratively, a portion of outer base 105 may be disposed in a portion of nosecone 110 wherein outer base distal end 106 may be disposed between nosecone distal end 111 and nosecone proximal end 112, e.g., a portion of outer base 105 may be disposed in a portion of nosecone 110 wherein nosecone proximal end 112 may be disposed between outer base distal end 106 and outer base proximal end 107.

In one or more embodiments, transitory element 100 may comprise a fixation mechanism distal housing 121, an inner lumen 122, an inner bore 123, a lock housing 125, and a pressure mechanism 130. Illustratively, pressure mechanism 130 may comprise a pressure mechanism distal end 131 and a pressure mechanism proximal end 132. In one or more embodiments, pressure mechanism 130 may be disposed between inner lumen 122 and hypodermic tube housing 113, e.g., pressure mechanism 130 may be disposed between inner lumen 122 and hypodermic tube housing 113 wherein pressure mechanism distal end 131 is adjacent to a proximal end of hypodermic tube housing 113 and pressure mechanism proximal end 132 is adjacent to a distal end of inner lumen 122. Illustratively, inner bore 123 may be disposed in pressure mechanism 130. In one or more embodiments, a portion of pressure mechanism 130 may be disposed in the plurality of radial projections 115. In one or more embodiments, pressure mechanism 130 may be configured to provide a force. Illustratively, pressure mechanism 130 may be configured to provide a constant or uniform force. In one or more embodiments, pressure mechanism 130 may be configured to provide a variable force. Illustratively, pressure mechanism 130 may comprise a spring or a coil. In one or more embodiments, pressure mechanism 130 may comprise a spring having a spring constant in a range of 5.25 to 11.0 pounds per inch, e.g., pressure mechanism 130 may comprise a spring having a spring constant of 7.5 pounds per inch. Illustratively, pressure mechanism 130 may comprise a spring having a spring constant less than 5.25 pounds per inch or greater than 11.0 pounds per inch. In one or more embodiments, pressure mechanism 130 may comprise a spring having a spring constant in a range of 7.7 to 15.8 pounds per inch, e.g., pressure mechanism 130 may comprise a spring having a spring constant of 9.5 pounds per inch. Illustratively, pressure mechanism 130 may comprise a spring having a spring constant less than 7.7 pounds per inch or greater than 15.8 pounds per inch. In one or more embodiments, pressure mechanism 130 may comprise a pneumatic system. Illustratively, lock housing 125 may be disposed in outer base aperture 108, e.g., lock housing 125 may be configured to actuate within outer base aperture 108. In one or more embodiments, pressure mechanism 130 may be configured to provide a force that resists an actuation of lock housing 125 within outer base aperture 108. Illustratively, pressure mechanism 130 may be configured to provide a force that facilitates an actuation of lock housing 125 within outer base aperture 108.

In one or more embodiments, fixation mechanism distal receptacle 120 may be configured to extend a distance from outer base proximal end 107. Illustratively, fixation mechanism distal receptacle 120 may be configured to extend a distance from outer base proximal end 107 in a range of 0.02 to 0.06 inches, e.g., fixation mechanism distal receptacle 120 may be configured to extend a distance from outer base proximal end 107 of 0.045 inches. In one or more embodiments, fixation mechanism distal receptacle 120 may be configured to extend a distance from outer base proximal end 107 of less than 0.02 inches or greater than 0.06 inches. Illustratively, fixation mechanism distal housing 121 may be disposed in fixation mechanism distal receptacle 120. In one or more embodiments, inner lumen 122 may be disposed between fixation mechanism distal housing 121 and pressure mechanism 130. Illustratively, inner lumen 122 may be disposed between nosecone proximal end 112 and outer base proximal end 107. In one or more embodiments, a portion of lock housing 125 may be disposed in a portion of inner lumen 122. Illustratively, outer base aperture 108 may be disposed between fixation mechanism distal housing 121 and pressure mechanism proximal end 132. In one or more embodiments, lock housing 125 may be disposed between fixation mechanism distal housing 121 and pressure mechanism proximal end 132. Illustratively, hypodermic tube housing 113 may be disposed between inner bore 123 and nosecone distal end 111.

In one or more embodiments, transitory element 100 may be manufactured from a material configured to deform if transitory element 100 is sterilized in a medical autoclave, e.g., transitory element 100 may be manufactured from a material configured to permanently deform if transitory element 100 is sterilized in a medical autoclave. Illustratively, transitory element 100 may be manufactured from a material having a melting point below a temperature parameter for a steam sterilization cycle, e.g., transitory element 100 may be manufactured from a material having a melting point below a temperature parameter for a gravity-displacement steam sterilization cycle, a dynamic-air-removal steam sterilization cycle, etc. In one or more embodiments, transitory element 100 may be manufactured from a material having a melting point below 140.0 degrees Fahrenheit. Illustratively, transitory element 100 may be manufactured from a material having a melting point in a range of 158.0 to 212.0 degrees Fahrenheit, e.g., transitory element 100 may be manufactured from a material having a melting point of 160.0 degrees Fahrenheit. In one or more embodiments, transitory element 100 may be manufactured from a material having a melting point of less than 158.0 degrees Fahrenheit or greater than 212.0 degrees Fahrenheit. In one or more embodiments, transitory element 100 may be manufactured from a material having a melting point below 250.0 degrees Fahrenheit. Illustratively, transitory element 100 may be manufactured from a material having a melting point below 270.0 degrees Fahrenheit. In one or more embodiments, transitory element 100 may be manufactured from a material having a melting point below 275.0 degrees Fahrenheit.

Illustratively, transitory element 100 may be manufactured from a material configured to temporarily deform if transitory element 100 is sterilized in a medical autoclave, e.g., transitory element 100 may be manufactured from a material configured to absorb water in a medical autoclave. In one or more embodiments, an absorption of water may be configured to deform transitory element 100, e.g., an absorption of water may be configured to cause transitory element 100 to expand. Illustratively, transitory element 100 may be manufactured from a porous material configured to facilitate a deformation of transitory element 100 if transitory element 100 is sterilized in a medical autoclave. In one or more embodiments, transitory element 100 may be manufactured with one or more cavities configured to facilitate a deformation of transitory element 100 if transitory element 100 is sterilized in a medical autoclave. Illustratively, transitory element 100 may be manufactured from any suitable material, e.g., polymers, metals, metal alloys, etc., or from any combination of suitable materials. In one or more embodiments, transitory element 100 may be manufactured by a 3D printing process. For example, transitory element 100 may be manufactured by selective laser sintering, selective heat sintering, selective laser melting, electron-beam melting, direct metal laser sintering, electron beam freeform fabrication, etc. Illustratively, transitory element 100 may be manufactured by injection molding.

In one or more embodiments, transitory element 100 may be manufactured from poly(acrylamide), poly(acrylic acid), poly(adipic anhydride), poly(7-aminoenanthic acid), poly(12-aminolauric acid), poly(11-aminoundecanoic acid), poly(azelaic anhydride), poly[1,3-butadiene(1,4-)-alt-methacrylonitrile], poly[1,3-butadiene(1,4-)-alt-methyl methacrylate], poly(butadiene oxide), poly(caprylaldehyde), poly(1,4-cyclohexylenedimethylene azelate), poly(1,4-cyclohexylenedimethylene dodecanedioate), poly(1,4-cyclohexylenedimethylene glutarate), poly(1,4-cyclohexylenedimethylene p-phenylenediacetate), poly(1,4-cyclohexylenedimethylene pimelate), poly(1,4-cyclohexylenedimethylene sebacate), poly(1,4-cyclohexylenedimethylene suberate), poly(cyclohexylidenethiohexamethylene sulfide), poly(cyclopropylenedimethylene piperazinediurethane), poly(cyclopropylidenedimethylene oxide), poly(decamethylene), poly(decamethylene carbonate), poly[(decamethylenedioxy)-dihexamethylene oxide], poly(decamethylene disulfide), poly(decamethylenedithioethylene disulfide), poly(decamethylenedithiohexamethylene disulfide), poly(decamethylene dithioladipate), poly(decamethylenedithiotetramethylene disulfide), poly(decamethylene pimelate), poly(decamethylene fumaramide), poly(decamethylene glutaramide), poly(decamethylene isophthalate), poly(decamethylene malonate), poly(decamethylene oxydiacetate), poly(decamethyleneoxymethylene oxide), poly(decamethylene succinate), poly(decamethylene sulfide), poly(decamethylene thiodivalerate), poly(decamethylenethiohexamethylene sulfide), poly(divinylbenzal), poly(dodecamethylene), poly(dodecanedioic anhydride), poly(eicosamethylene adipate), poly(eicosamethylene azelate), poly(eicosamethylene glutarate), poly(eicosamethylene isophthalate), poly(eicosamethylene malonate), poly(eicosamethylene oxalate), poly(eicosamethylene oxydiacetate), poly(eicosamethylene phthalate), poly(eicosamethylene pimelate), poly(eicosamethylene sebacate), poly(eicosamethylene suberate), poly(eicosamethylene succinate), poly(eicosamethylene thiodivalerate), poly[ethylene p-(carboxyphenoxy)-butyrate], poly[ethylene p-(carboxyphenoxy)-caproate], poly[ethylene p-(carboxyphenoxy)-heptanoate], poly[ethylene p-(carboxyphenoxy)-undecanoate], poly[ethylene p-(carboxyphenoxy)-valerate], poly(ethylene 2,2'-dibenzoate), poly[(ethylenedioxy)-diethylene 2,2'-dibenzoate], poly(ethylene 2,2'-dibenzoate), poly[(ethylenedioxy)-diethylene 3,3'-dibenzoate], poly[(ethylenedioxy)-diethylene isophthalate], poly[(ethylenedioxy)-diethylene sebacate], poly[(ethylenedioxy)-diethylene thiodivalerate], poly(ethylene disiloxanylenedi-propionamide), poly[(ethylenedithio)-diacetic anhydride], poly[(ethylenedithio)-dipropionic anhydride], poly(ethylene dithionisophthalate), poly(ethelene dithiotetra-methylene disulfide), poly(ethylene fumaramide), poly(ethylene glutarate), poly(ethylene 2,4-hexadienediamide), poly(ethylene phthalate), poly(ethylene sulfonyldivalerate), poly(ethylene terephthalate), poly(heptamethylene), poly(hexamethylene azelate), poly(hexamethylene carbonate), poly[hexamethylene p-(carboxyphenoxy)-acetate], poly[hexamethylene p-(carboxyphenoxy)-caproate], poly[hexamethylene p-(carboxyphenoxy)-undecanoate], poly[hexamethylene p-(carboxyphenoxy)-valerate], poly(hexamethylene isophthalate), poly[hexamethylene (methylene-2,5-tetrahydrofuran)-dicarboxamide], poly(hexamethylene octadecanediamide), poly(hexamethylene oxydiacetate), poly(hexamethylene 4,4'-oxydibenzoate), poly(hexamethylene pimelate), poly(hexamethylene succinate), poly(hexamethylene thiodivalerate), poly(hexamethylenethiooentamethylene sulfide), poly(hexamethylenethiotetramethylene sulfide), poly(hexenamer), etc. Illustratively, transitory element 100 may be manufactured from any substituted polymers of poly(acrylamide), poly(acrylic acid), poly(adipic anhydride), poly(7-aminoenanthic acid), poly(12-aminolauric acid), poly(11-aminoundecanoic acid), poly(azelaic anhydride), poly[1,3-butadiene(1,4-)-alt-methacrylonitrile], poly[1,3-butadiene(1,4-)-alt-methyl methacrylate], poly(butadiene oxide), poly(caprylaldehyde), poly(1,4-cyclohexylenedimethylene azelate), poly(1,4-cyclohexylenedimethylene dodecanedioate), poly(1,4-cyclohexylenedimethylene glutarate), poly(1,4-cyclohexylenedimethylene p-phenylenediacetate), poly(1,4-cyclohexylenedimethylene pimelate), poly(1,4-cyclohexylenedimethylene sebacate), poly(1,4-cyclohexylenedimethylene suberate), poly(cyclohexylidenethiohexamethylene sulfide), poly(cyclopropylenedimethylene piperazinediurethane), poly(cyclopropylidenedimethylene oxide), poly(decamethylene), poly(decamethylene carbonate), poly[(decamethylenedioxy)-dihexamethylene oxide], poly(decamethylene disulfide), poly(decamethylenedithioethylene disulfide), poly(decamethylenedithiohexamethylene disulfide), poly(decamethylene dithioladipate), poly(decamethylenedithiotetramethylene disulfide), poly(decamethylene pimelate), poly(decamethylene fumaramide), poly(decamethylene glutaramide), poly(decamethylene isophthalate), poly(decamethylene malonate), poly(decamethylene oxydiacetate), poly(decamethyleneoxymethylene oxide), poly(decamethylene succinate), poly(decamethylene sulfide), poly(decamethylene thiodivalerate), poly(decamethylenethiohexamethylene sulfide), poly(divinylbenzal), poly(dodecamethylene), poly(dodecanedioic anhydride), poly(eicosamethylene adipate), poly(eicosamethylene azelate), poly(eicosamethylene glutarate), poly(eicosamethylene isophthalate), poly(eicosamethylene malonate), poly(eicosamethylene oxalate), poly(eicosamethylene oxydiacetate), poly(eicosamethylene phthalate), poly(eicosamethylene pimelate), poly(eicosamethylene sebacate), poly(eicosamethylene suberate), poly(eicosamethylene succinate), poly(eicosamethylene thiodivalerate), poly[ethylene p-(carboxyphenoxy)-butyrate], poly[ethylene p-(carboxyphenoxy)-caproate], poly[ethylene p-(carboxyphenoxy)-heptanoate], poly[ethylene p-(carboxyphenoxy)-undecanoate], poly[ethylene p-(carboxyphenoxy)-valerate], poly(ethylene 2,2'-dibenzoate), poly[(ethylenedioxy)-diethylene 2,2'-dibenzoate], poly(ethylene 2,2'-dibenzoate), poly[(ethylenedioxy)-diethylene 3,3'-dibenzoate], poly[(ethylenedioxy)-diethylene isophthalate], poly[(ethylenedioxy)-diethylene sebacate], poly[(ethylenedioxy)-diethylene thiodivalerate], poly(ethylene disiloxanylenedi-propionamide), poly[(ethylenedithio)-diacetic anhydride], poly[(ethylenedithio)-dipropionic anhydride], poly(ethylene dithionisophthalate), poly(ethelene dithiotetra-methylene disulfide), poly(ethylene fumaramide), poly(ethylene glutarate), poly(ethylene 2,4-hexadienediamide), poly(ethylene phthalate), poly(ethylene sulfonyldivalerate), poly(ethylene terephthalate), poly(heptamethylene), poly(hexamethylene azelate), poly(hexamethylene carbonate), poly[hexamethylene p-(carboxyphenoxy)-acetate], poly[hexamethylene p-(carboxyphenoxy)-caproate], poly[hexamethylene p-(carboxyphenoxy)-undecanoate], poly[hexamethylene p-(carboxyphenoxy)-valerate], poly(hexamethylene isophthalate), poly[hexamethylene (methylene-2,5-tetrahydrofuran)-dicarboxamide], poly(hexamethylene octadecanediamide), poly(hexamethylene oxydiacetate), poly(hexamethylene 4,4'-oxydibenzoate), poly(hexamethylene pimelate), poly(hexamethylene succinate), poly(hexamethylene thiodivalerate), poly(hexamethylenethiooentamethylene sulfide), poly(hexamethylenethiotetramethylene sulfide), poly(hexenamer), etc.

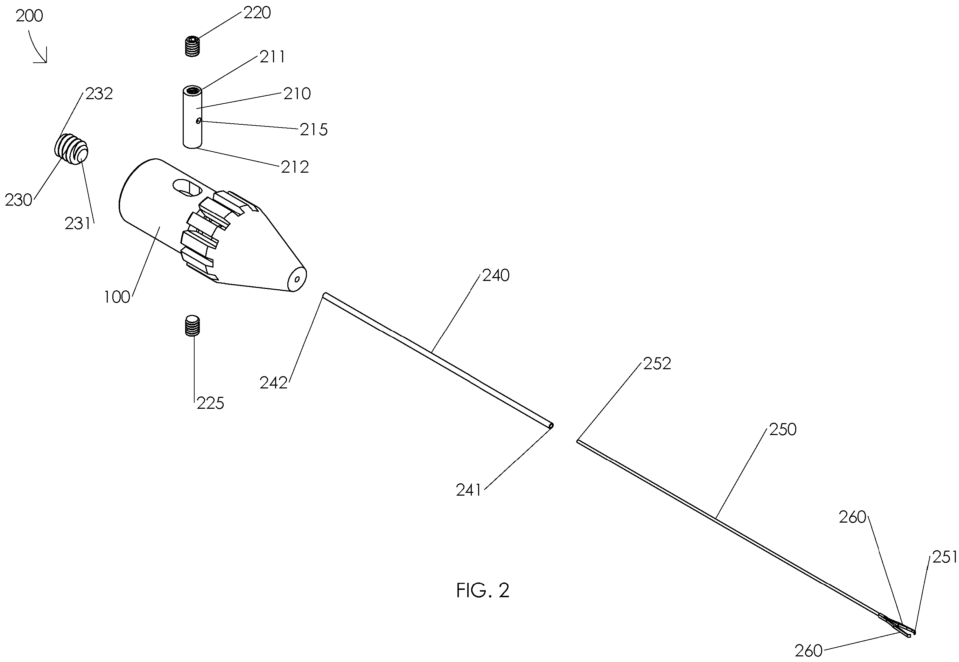

FIG. 2 is a schematic diagram illustrating an exploded view of an instrument tip assembly 200. Illustratively, an instrument tip assembly 200 may comprise a transitory element 100, a lock 210, a superior fixation mechanism 220, an inferior fixation mechanism 225, a fixation mechanism 230, a hypodermic tube 240, and a blank 250. In one or more embodiments, lock 210 may comprise a lock superior end 211 and a lock inferior end 212. Illustratively, lock 210 may comprise a blank housing 215, e.g., lock 210 may comprise a blank housing 215 disposed between lock superior end 211 and lock inferior end 212. In one or more embodiments, superior fixation mechanism 220 may comprise a setscrew, a magnet, an adhesive, a weld, etc. Illustratively, inferior fixation mechanism 225 may comprise a setscrew, a magnet, an adhesive, a weld, etc. In one or more embodiments, fixation mechanism 230 may comprise a fixation mechanism distal end 231 and a fixation mechanism proximal end 232. Illustratively, fixation mechanism 230 may comprise a setscrew, a magnet, an adhesive, a weld, etc. In one or more embodiments, hypodermic tube 240 may comprise a hypodermic tube distal end 241 and a hypodermic tube proximal end 242. Illustratively, blank 250 may comprise a blank distal end 251 and a blank proximal end 252. In one or more embodiments, blank 250 may comprise one or more instrument jaws 260, e.g., blank 250 may comprise a pair of instrument jaws 260. Illustratively, blank 250 may comprise a plurality of instrument jaws 260, e.g., blank 250 may comprise two instrument jaws 260, three instrument jaws 260, four instrument jaws 260, five instrument jaws 260, six instrument jaws 260, etc. In one or more embodiments, instrument jaws 260 may comprise forceps jaws. Illustratively, instrument jaws 260 may comprise scissors jaws.

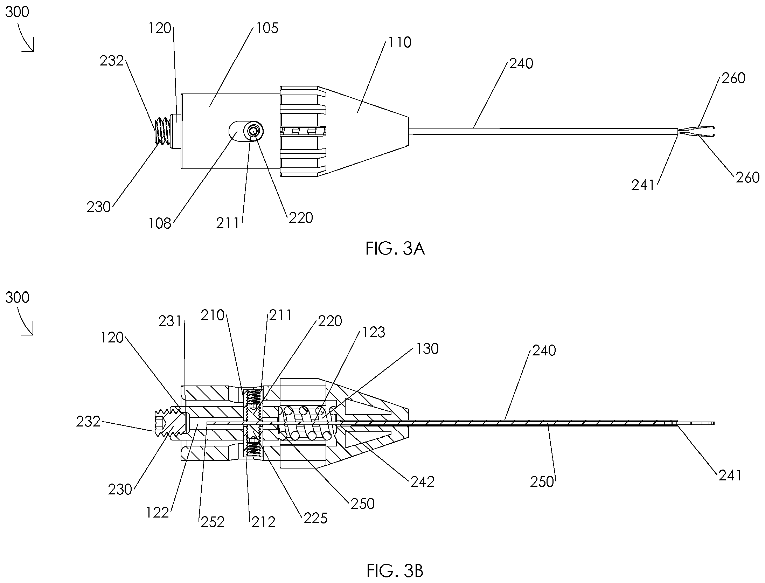

FIGs. 3A and 3B are schematic diagrams illustrating an assembled instrument tip 300. FIG. 3A illustrates a superior view of an assembled instrument tip 300. FIG. 3B illustrates a cross-sectional view in a sagittal plane of an assembled instrument tip 300. Illustratively, a portion of hypodermic tube 240 may be disposed in a portion of nosecone 110, e.g., hypodermic tube proximal end 242 may be disposed in a portion of nosecone 110. In one or more embodiments, a portion of hypodermic tube 240 may be fixed within a portion of nosecone 110, e.g., a portion of hypodermic tube 240 may be fixed within a portion of nosecone 110 by an interference fit, an adhesive, a setscrew, a weld, etc. Illustratively, a portion of hypodermic tube 240 may be disposed in hypodermic tube housing 113, e.g., hypodermic tube proximal end 242 may be disposed in hypodermic tube housing 113 wherein hypodermic tube distal end 241 extends from transitory element distal end 101. In one or more embodiments, a portion of hypodermic tube 240 may be fixed within hypodermic tube housing 113, e.g., a portion of hypodermic tube 240 may be fixed within hypodermic tube housing 113 by an interference fit, an adhesive, a setscrew, a weld, etc. Illustratively, lock 210 may be disposed in a portion of transitory element 100, e.g., lock 210 may be disposed in outer base aperture 108. In one or more embodiments, lock 210 may be disposed in outer base aperture 108 wherein lock 210 may be disposed in lock housing 125. Illustratively, lock 210 may be disposed in lock housing 125 wherein blank housing 215 is oriented to align with hypodermic tube housing 113, e.g., lock 210 may be disposed in lock housing 125 wherein blank housing 215 is disposed within inner lumen 122. In one or more embodiments, lock 210 may be fixed in lock housing 125, e.g., lock 210 may be fixed in lock housing 125 by an interference fit, an adhesive, a setscrew, a weld, etc.

Illustratively, blank 250 may be disposed in hypodermic tube 240, e.g., blank 250 may be disposed in hypodermic tube 240 wherein blank distal end 251 extends from hypodermic tube distal end 241. In one or more embodiments, blank 250 may be disposed in hypodermic tube 240, nosecone 110, hypodermic tube housing 113, inner bore 123, pressure mechanism 130, outer base 105, inner lumen 122, outer base aperture 108, lock housing 125, lock 210, and blank housing 215. Illustratively, superior fixation mechanism 220 may be disposed in lock 210, e.g., superior fixation mechanism 220 may be disposed in lock superior end 211. In one or more embodiments, inferior fixation mechanism 225 may be disposed in lock 210, e.g., inferior fixation mechanism 225 may be disposed in lock inferior end 212. Illustratively, a portion of blank 250 may be disposed between superior fixation mechanism 220 and inferior fixation mechanism 225 within lock 210. In one or more embodiments, superior fixation mechanism 220 and inferior fixation mechanism 225 may be configured to fix a portion of blank 250 within lock 210. For example, superior fixation mechanism 220 may comprise a first setscrew and inferior fixation mechanism 225 may comprise a second setscrew. Illustratively, a portion of blank 250 may be fixed in lock 210 by an interference fit, an adhesive, a setscrew, a weld, etc.

In one or more embodiments, a portion of fixation mechanism 230 may be disposed in fixation mechanism distal receptacle 120, e.g., fixation mechanism distal end 231 may be disposed in fixation mechanism distal receptacle 120. Illustratively, a first portion of fixation mechanism 230 may be disposed in fixation mechanism distal receptacle 120 wherein a second portion of fixation mechanism 230 extends from transitory element proximal end 102, e.g., fixation mechanism distal end 231 may be disposed in fixation mechanism distal receptacle 120 wherein fixation mechanism proximal end 232 extends from transitory element proximal end 102. In one or more embodiments, a portion of fixation mechanism 230 may be disposed in fixation mechanism distal housing 121. Illustratively, fixation mechanism distal end 231 may be disposed in fixation mechanism distal housing 121 wherein fixation mechanism proximal end 232 extends from transitory element proximal end 102. In one or more embodiments, fixation mechanism distal end 231 may be disposed in fixation mechanism distal housing 121 wherein fixation mechanism proximal end 232 extends a distance from transitory element proximal end 102 in a range of 0.065 to 0.125 inches, e.g., fixation mechanism distal end 231 may be disposed in fixation mechanism distal housing 121 wherein fixation mechanism proximal end 232 extends a distance from transitory element proximal end 102 of 0.094 inches. Illustratively, fixation mechanism distal end 231 may be disposed in fixation mechanism distal housing 121 wherein fixation mechanism proximal end 232 extends a distance from transitory element proximal end 102 of less than 0.065 inches or greater than 0.125 inches. In one or more embodiments, a portion of fixation mechanism 230 may be fixed in fixation mechanism distal housing 121, e.g., fixation mechanism distal end 231 may be fixed in fixation mechanism distal housing 121. Illustratively, a portion of fixation mechanism 230 may be fixed in fixation mechanism distal housing 121 by an interference fit, an adhesive, a magnetic field, a weld, a threading, etc.

In one or more embodiments, assembled instrument tip 300 may be a single-use instrument, e.g., assembled instrument tip 300 may be intended for only one use in a surgery. Illustratively, assembled instrument tip 300 may be sterilized after manufacturing but prior to shipment of assembled instrument tip 300 to a user, e.g., assembled instrument tip 300 may be sterilized by ethylene oxide after manufacturing but prior to shipment of assembled instrument tip 300 to a user. In one or more embodiments, one or more properties of assembled instrument tip 300 may be configured to prevent a user from using a sterile assembled instrument tip 300 in a first surgical procedure causing the assembled instrument tip 300 to become non-sterile, sterilizing the assembled instrument tip 300, and using the sterile assembled instrument tip 300 in a second surgical procedure. Illustratively, transitory element 100 may be manufactured from a material configured to deform if transitory element 100 is sterilized in a medical autoclave. In one or more embodiments, transitory element 100 may be manufactured from a material configured to retain ethylene oxide, e.g., transitory element 100 may be manufactured from a material having a degree of crystallinity greater than 60.0 percent. For example, transitory element 100 may be manufactured from a material having a degree of crystallinity greater than 70.0 percent. In one or more embodiments, transitory element 100 may be manufactured from a material having a degree of crystallinity in a range of 60.0 to 80.0 percent, e.g., transitory element 100 may be manufactured from a material having a degree of crystallinity of 75.0 percent. Illustratively, transitory element 100 may be manufactured from a material having a degree of crystallinity of less than 60.0 percent or greater than 80.0 percent. In one or more embodiments, transitory element 100 may be manufactured from a material configured to retain less than 4.0 milligrams of ethylene oxide after a first sterilization by ethylene oxide and configured to retain more than 4.0 milligrams of ethylene oxide after a second sterilization by ethylene oxide. For example, transitory element 100 may be manufactured from polyoxymethylene, polytetrafluoroethylene, isotactic polypropylene, high-density polyethylene, etc. In one or more embodiments, transitory element 100 may be manufactured from a material configured to degrade if transitory element 100 is sterilized by plasma sterilization, e.g., transitory element 100 may be manufactured by a material configured to cross-link in plasma sterilization.

Illustratively, fixation mechanism 230 may be manufactured from a material configured to deform if fixation mechanism 230 is sterilized in a medical autoclave. In one or more embodiments, fixation mechanism 230 may be manufactured from a material having a melting point below 140.0 degrees Fahrenheit. Illustratively, fixation mechanism 230 may be manufactured from a material having a melting point in a range of 158.0 to 212.0 degrees Fahrenheit, e.g., fixation mechanism 230 may be manufactured from a material having a melting point of 160.0 degrees Fahrenheit. In one or more embodiments, fixation mechanism 230 may be manufactured from a material having a melting point of less than 158.0 degrees Fahrenheit or greater than 212.0 degrees Fahrenheit. Illustratively, lock 210 may be manufactured from a material configured to deform if lock 210 is sterilized in a medical autoclave. In one or more embodiments, lock 210 may be manufactured from a material having a melting point below 140.0 degrees Fahrenheit. Illustratively, lock 210 may be manufactured from a material having a melting point in a range of 158.0 to 212.0 degrees Fahrenheit, e.g., lock 210 may be manufactured from a material having a melting point of 160.0 degrees Fahrenheit. In one or more embodiments, lock 210 may be manufactured from a material having a melting point of less than 158.0 degrees Fahrenheit or greater than 212.0 degrees Fahrenheit. Illustratively, hypodermic tube 240 may be manufactured from a material configured to deform if hypodermic tube 240 is sterilized in a medical autoclave. In one or more embodiments, hypodermic tube 240 may be manufactured from a material having a melting point below 140.0 degrees Fahrenheit. Illustratively, hypodermic tube 240 may be manufactured from a material having a melting point in a range of 158.0 to 212.0 degrees Fahrenheit, e.g., hypodermic tube 240 may be manufactured from a material having a melting point of 160.0 degrees Fahrenheit. In one or more embodiments, hypodermic tube 240 may be manufactured from a material having a melting point of less than 158.0 degrees Fahrenheit or greater than 212.0 degrees Fahrenheit. Illustratively, blank 250 may be manufactured from a material configured to deform if blank 250 is sterilized in a medical autoclave. In one or more embodiments, blank 250 may be manufactured from a material having a melting point below 140.0 degrees Fahrenheit. Illustratively, blank 250 may be manufactured from a material having a melting point in a range of 158.0 to 212.0 degrees Fahrenheit, e.g., blank 250 may be manufactured from a material having a melting point of 160.0 degrees Fahrenheit. In one or more embodiments, blank 250 may be manufactured from a material having a melting point of less than 158.0 degrees Fahrenheit or greater than 212.0 degrees Fahrenheit.

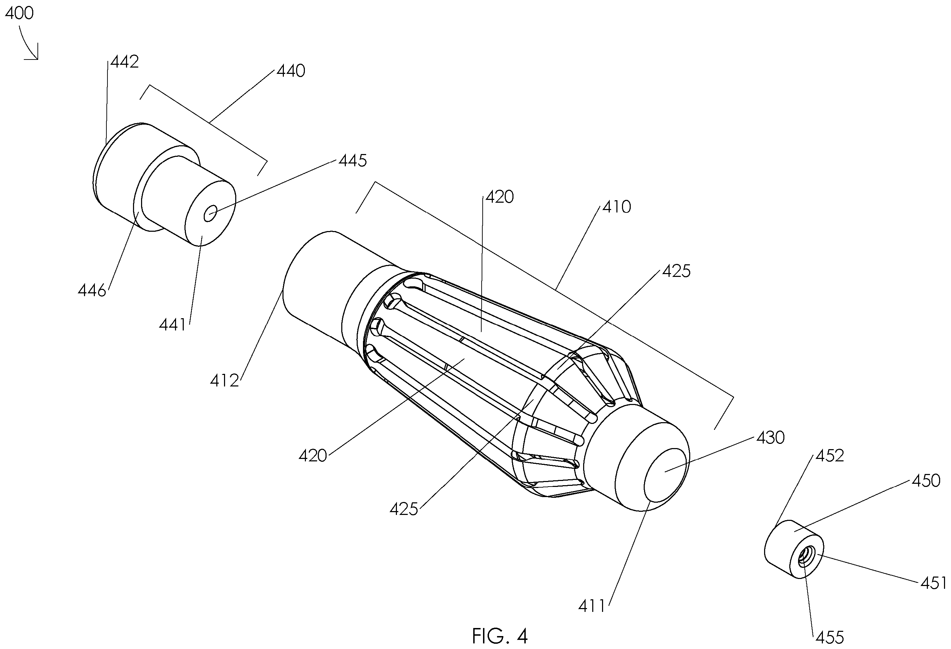

FIG. 4 is a schematic diagram illustrating an exploded view of an instrument handle assembly 400. Illustratively, an instrument handle assembly 400 may comprise an actuation structure 410, a handle base 440, and a fixation mechanism proximal receptacle 450. In one or more embodiments, an actuation structure 410 may comprise an actuation structure distal end 411 and an actuation structure proximal end 412. Illustratively, actuation structure 410 may comprise an instrument tip housing 430. In one or more embodiments, instrument tip housing 430 may be configured to house assembled instrument tip 300. Illustratively, actuation structure 410 may comprise a plurality of actuation arms 420. In one or more embodiments, each actuation arm 420 may comprise at least one extension joint 425. In one or more embodiments, actuation structure 410 may comprise a shape memory material configured to project actuation structure distal end 411 a first distance from actuation structure proximal end 412, e.g., when actuation structure 410 is fully decompressed. Illustratively, actuation structure 410 may comprise a shape memory material configured to project actuation structure distal end 411 a second distance from actuation structure proximal end 412, e.g., when actuation structure 410 is fully compressed. In one or more embodiments, the second distance from actuation structure proximal end 412 may be greater than the first distance from actuation structure proximal end 412. Actuation structure 410 may be manufactured from any suitable material, e.g., polymers, metals, metal alloys, etc., or from any combination of suitable materials. Illustratively, actuation structure 410 may be compressed by an application of a compressive force to actuation structure 410. In one or more embodiments, actuation structure 410 may be compressed by an application of one or more compressive forces located at one or more locations around an outer perimeter of actuation structure 410. Illustratively, the one or more locations may comprise any of a plurality of locations around the outer perimeter of actuation structure 410. For example, a surgeon may compress actuation structure 410 by squeezing actuation structure 410. Illustratively, the surgeon may compress actuation structure 410 by squeezing actuation structure 410 at any particular location of a plurality of locations around an outer perimeter of actuation structure 410. In one or more embodiments, actuation structure 410 may be compressed by an application of a compressive force to any one or more of the plurality of actuation arms 420. Illustratively, each actuation arm 420 may be configured to actuate independently. In one or more embodiments, each actuation arm 420 may be connected to one or more of the plurality of actuation arms 420 wherein an actuation of a particular actuation arm 420 may be configured to actuate every actuation arm 420 of the plurality of actuation arms 420. Illustratively, one or more actuation arms 420 may be configured to actuate in pairs or groups. For example, an actuation of a first actuation arm 420 may be configured to actuate a second actuation arm 420. In one or more embodiments, a compression of actuation structure 410, e.g., due to an application of a compressive force to a particular actuation arm 420, may be configured to actuate the particular actuation arm 420. Illustratively, an actuation of the particular actuation arm 420 may be configured to actuate every actuation arm 420 of the plurality of actuation arms 420. In one or more embodiments, an application of a compressive force to a particular actuation arm 420 may be configured to extend at least one extension joint 425 of the particular actuation arm 420.

Illustratively, handle base 440 may comprise a handle base distal end 441 and a handle base proximal end 442. In one or more embodiments, handle base 440 may comprise a handle base inner lumen 445. Illustratively, handle base 440 may comprise an actuation structure interface 446 configured to interface with a portion of actuation structure 410, e.g., handle base 440 may comprise an actuation structure interface 446 configured to interface with actuation structure proximal end 412. In one or more embodiments, fixation mechanism proximal receptacle 450 may comprise a fixation mechanism proximal receptacle distal end 451 and a fixation mechanism proximal receptacle proximal end 452. Illustratively, fixation mechanism proximal receptacle 450 may comprise a fixation mechanism proximal housing 455. In one or more embodiments, fixation mechanism proximal housing 455 may be configured to house a portion of fixation mechanism 230, e.g., fixation mechanism proximal housing 455 may be configured to house fixation mechanism proximal end 232.

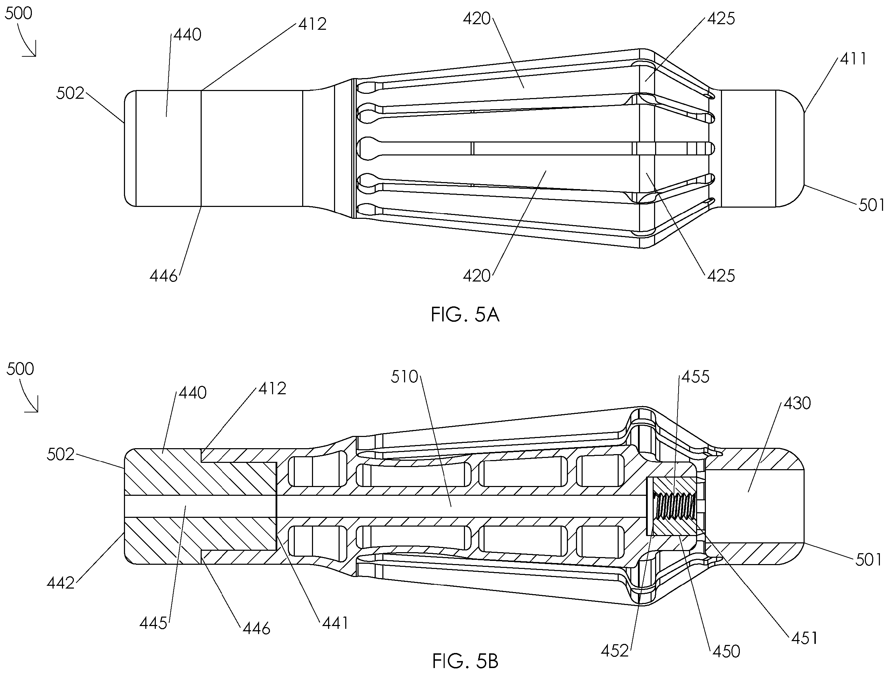

FIGs. 5A and 5B are schematic diagrams illustrating an assembled instrument handle 500. FIG. 5A illustrates a superior view of an assembled instrument handle 500. FIG. 5B illustrates a cross-sectional view in a sagittal plane of an assembled instrument handle 500. Illustratively, assembled instrument handle 500 may comprise an assembled instrument handle distal end 501 and an assembled instrument handle proximal end 502. In one or more embodiments, assembled instrument handle 500 may comprise a handle inner lumen 510. Illustratively, a portion of handle base 440 may be disposed in a portion of actuation structure 410, e.g., handle base distal end 441 may be disposed in actuation structure proximal end 412. In one or more embodiments, a portion of handle base 440 may be disposed in a portion of actuation structure 410 wherein actuation structure interface 446 is adjacent to actuation structure proximal end 412. Illustratively, a portion of handle base 440 may be disposed in a portion of actuation structure 410 wherein handle base inner lumen 445 is oriented to align with handle inner lumen 510. In one or more embodiments, a portion of handle base 440 may be disposed in actuation structure 410 wherein handle base proximal end 442 is assembled instrument handle proximal end 502. Illustratively, a portion of handle base 440 may be fixed in a portion of actuation structure 410, e.g., a portion of handle base 440 may be fixed in a portion of actuation structure 410 by an interference fit, an adhesive, a magnetic field, a weld, a threading, etc.

In one or more embodiments, fixation mechanism proximal receptacle 450 may be disposed in actuation structure 410, e.g., fixation mechanism proximal receptacle 450 may be disposed in actuation structure 410 wherein fixation mechanism proximal receptacle distal end 451 is disposed in actuation structure 410 and fixation mechanism proximal receptacle proximal end 452 is disposed in actuation structure 410. Illustratively, fixation mechanism proximal receptacle 450 may be disposed in actuation structure 410 wherein fixation mechanism proximal receptacle 450 is disposed between handle inner lumen 510 and instrument tip housing 430. In one or more embodiments, fixation mechanism proximal receptacle 450 may be disposed in actuation structure 410 wherein fixation mechanism proximal receptacle distal end 451 may be adjacent to a portion of instrument tip housing 430. Illustratively, fixation mechanism proximal receptacle 450 may be disposed in actuation structure 410 wherein fixation mechanism proximal receptacle proximal end 452 may be adjacent to a portion of handle inner lumen 510. In one or more embodiments, fixation mechanism proximal receptacle 450 may be fixed in actuation structure 410, e.g., fixation mechanism proximal receptacle 450 may be fixed in actuation structure 410 by an interference fit, an adhesive, a magnetic field, a weld, a threading, etc. In one or more embodiments, assembled instrument handle 500 may be manufactured from a material suitable for sterilization by a medical autoclave. Illustratively, assembled instrument handle 500 may be manufactured from a material, e.g., Nylon, configured to withstand exposure to temperatures, pressures, and ambient conditions present in a medical autoclave without degradation. For example, assembled instrument handle 500 may be configured to function normally after exposure in a temperature 275 degrees Fahrenheit. In one or more embodiments, assembled instrument handle 500 may be configured to be used in a surgical procedure and then sterilized by a medical autoclave at least three times. Illustratively, assembled instrument handle 500 may be configured to be used in a surgical procedure and then sterilized by a medical autoclave more than three times. In one or more embodiments, assembled instrument handle 500 may be configured to be used in a surgical procedure and then sterilized by a medical autoclave at least nine times. Illustratively, assembled instrument handle 500 may be configured to be used in a surgical procedure and then sterilized by a medical autoclave more than nine times.

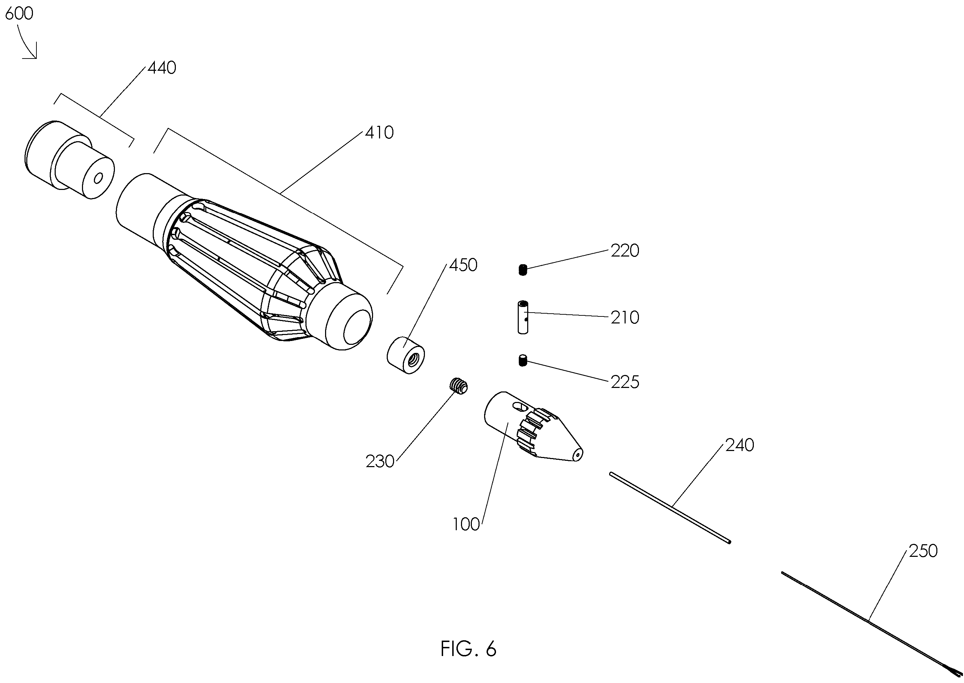

FIG. 6 is a schematic diagram illustrating an exploded view of an instrument assembly 600. Illustratively, an instrument assembly 600 may comprise a handle base 440, an actuation structure 410, a fixation mechanism proximal receptacle 450, a fixation mechanism 230, a transitory element 100, a lock 210, a superior fixation mechanism 220, an inferior fixation mechanism 225, a hypodermic tube 240, and a blank 250. In one or more embodiments, a portion of fixation mechanism 230 may be disposed in fixation mechanism proximal receptacle 450, e.g., fixation mechanism proximal end 232 may be disposed in fixation mechanism proximal receptacle 450. Illustratively, a first portion of fixation mechanism 230 may be disposed in fixation mechanism proximal receptacle 450 wherein a second portion of fixation mechanism 230 may be disposed in fixation mechanism distal receptacle 120, e.g., fixation mechanism proximal end 232 may be disposed in fixation mechanism proximal receptacle 450 and fixation mechanism distal end 231 may be disposed in fixation mechanism distal receptacle 120. In one or more embodiments, a portion of fixation mechanism 230 may be disposed in fixation mechanism proximal housing 455, e.g., fixation mechanism proximal end 232 may be disposed in fixation mechanism proximal housing 455. Illustratively, a first portion of fixation mechanism 230 may be disposed in fixation mechanism proximal housing 455 wherein a second portion of fixation mechanism 230 may be disposed in fixation mechanism distal housing 121, e.g., fixation mechanism proximal end 232 may be disposed in fixation mechanism proximal housing 455 and fixation mechanism distal end 321 may be disposed in fixation mechanism distal housing 121. In one or more embodiments, a portion of fixation mechanism 230 may be fixed in fixation mechanism proximal housing 455, e.g., fixation mechanism proximal end 232 may be fixed in fixation mechanism proximal housing 455. Illustratively, a portion of fixation mechanism 230 may be fixed in fixation mechanism proximal housing 455 by an interference fit, an adhesive, a magnetic field, a weld, a threading, etc. In one or more embodiments, a first portion of fixation mechanism 230 may be fixed in fixation mechanism proximal housing 455 and a second portion of fixation mechanism 230 may be fixed in fixation mechanism distal housing 121, e.g., fixation mechanism proximal end 232 may be fixed in fixation mechanism proximal housing 455 and fixation mechanism distal end 231 may be fixed in fixation mechanism distal housing 121.

Illustratively, a portion of transitory element 100 may be disposed in a portion of actuation structure 410, e.g., a portion of outer base 105 may be disposed in instrument tip housing 430. In one or more embodiments, fixation mechanism 230 may be configured to fix a portion of transitory element 100 in a portion of actuation structure 410, e.g., fixation mechanism 230 may be configured to fix a portion of outer base 105 in instrument tip housing 430. Illustratively, fixation mechanism 230 may comprise a setscrew configured to screw into fixation mechanism distal housing 121 and fixation mechanism proximal housing 455. In one or more embodiments, fixation mechanism 230 may be permanently fixed in fixation mechanism distal housing 121, e.g., fixation mechanism distal end 231 may be fixed in distal housing 121 wherein removing fixation mechanism distal end 231 from distal housing 121 may be configured to damage a portion of transitory element 100. Illustratively, fixation mechanism distal end 231 may be permanently fixed in distal housing 121 wherein removing fixation mechanism distal end 231 from distal housing 121 may be configured to damage fixation mechanism distal receptacle 120. In one or more embodiments, fixation mechanism 230 may be temporarily fixed in fixation mechanism proximal housing 455, e.g., fixation mechanism proximal end 232 may be fixed in fixation mechanism proximal housing 455 wherein fixation mechanism proximal end 232 is removable from fixation mechanism proximal housing 455. Illustratively, a first fixation mechanism proximal end 232 may be temporarily fixed in fixation mechanism proximal housing 455 wherein the first fixation mechanism proximal end 232 may be removed from fixation mechanism proximal housing 455 and a second fixation mechanism proximal end 232 may be inserted in fixation mechanism proximal housing 455. In one or more embodiments, a first fixation mechanism proximal end 232 may be temporarily fixed in fixation mechanism proximal housing 455 wherein the first fixation mechanism proximal end 232 may be removed from fixation mechanism proximal housing 455 and a second fixation mechanism proximal end 232 may be temporarily fixed in fixation mechanism proximal housing 455.

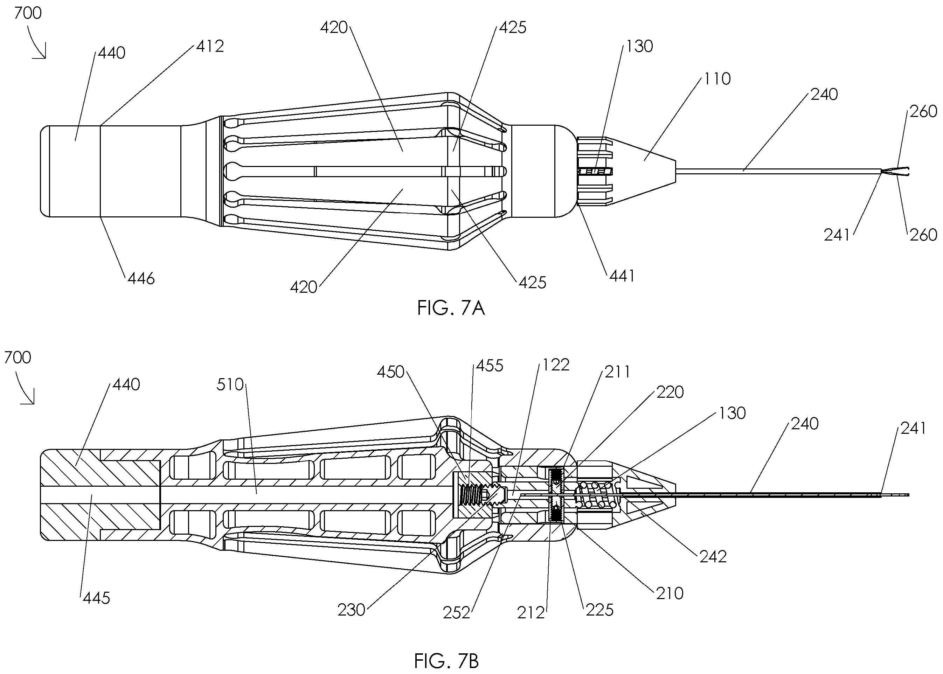

FIGs. 7A and 7B are schematic diagrams illustrating an instrument with open jaws 700. FIG. 7A illustrates a superior view of an instrument with open jaws 700. FIG. 7B illustrates a cross-sectional view in a sagittal plane of an instrument with open jaws 700. Illustratively, assembled instrument handle 500 and assembled instrument tip 300 may comprise an instrument with open jaws 700 when fixation mechanism proximal end 232 is temporarily fixed in fixation mechanism proximal housing 455. In one or more embodiments, assembled instrument handle 500 and assembled instrument tip 300 may comprise an instrument with open jaws 700 when a first instrument jaw 260 is fully separated from a second instrument jaw 260. Illustratively, assembled instrument handle 500 and assembled instrument tip 300 may comprise an instrument with open jaws 700 when actuation structure 210 is fully decompressed. In one or more embodiments, assembled instrument handle 500 and assembled instrument tip 300 may comprise an instrument with open jaws 700 when hypodermic tube 240 is fully retracted relative to blank 250. Illustratively, assembled instrument handle 500 and assembled instrument tip 300 may comprise an instrument with open jaws 700 when nosecone 110 is fully retracted relative to outer base 105. In one or more embodiments, assembled instrument handle 500 and assembled instrument tip 300 may comprise an instrument with open jaws 700 when pressure mechanism 130 is fully compressed.

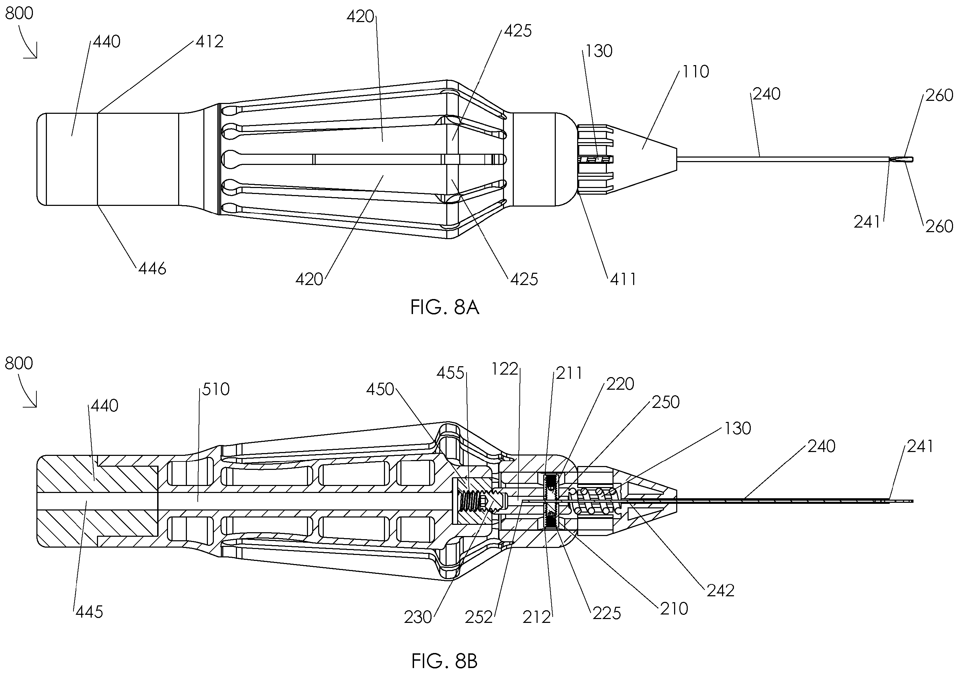

FIGs. 8A and 8B are schematic diagrams illustrating an instrument with partially closed jaws 800. FIG. 8A illustrates a superior view of an instrument with partially closed jaws 800. FIG. 8B illustrates a cross-sectional view in a sagittal plane of an instrument with partially closed jaws 800. Illustratively, a compression of actuation structure 410 may be configured to gradually transition assembled instrument handle 500 and assembled instrument tip 300 from an instrument with open jaws 700 to an instrument with partially closed jaws 800. In one or more embodiments, a compression of actuation structure 410 may be configured to extend actuation structure distal end 411 relative to actuation structure proximal end 412. Illustratively, an extension of actuation structure distal end 411 relative to actuation structure proximal end 412 may be configured to extend nosecone 110 relative to handle base 105. In one or more embodiments, an extension of nosecone 110 relative to handle base 105 may be configured to extend hypodermic tube 240 relative to blank 250. Illustratively, an extension of hypodermic tube 240 relative to blank 250 may be configured to extend hypodermic tube distal end 241 over a portion of a first instrument jaw 260 and over a portion of a second instrument jaw 260. In one or more embodiments, an extension of hypodermic tube distal end 241 over a portion of a first instrument jaw 260 and over a portion of a second instrument jaw 260 may be configured to reduce a separation distance between the first instrument jaw 260 and the second instrument jaw 260 until assembled instrument handle 500 and assembled instrument tip 300 comprise an instrument with partially closed jaws 800. Illustratively, an extension of nosecone 110 relative to handle base 105 may be configured to expand pressure mechanism 130, e.g., an extension of nosecone 110 relative to handle base 105 may be configured to extend pressure mechanism distal end 131 relative to pressure mechanism proximal end 132. In one or more embodiments, pressure mechanism 130 may be configured to provide a force that resists an extension of nosecone 110 relative to handle base 105, e.g., pressure mechanism 130 may be configured to provide a force that facilitates a retraction of nosecone 110 relative to handle base 105.

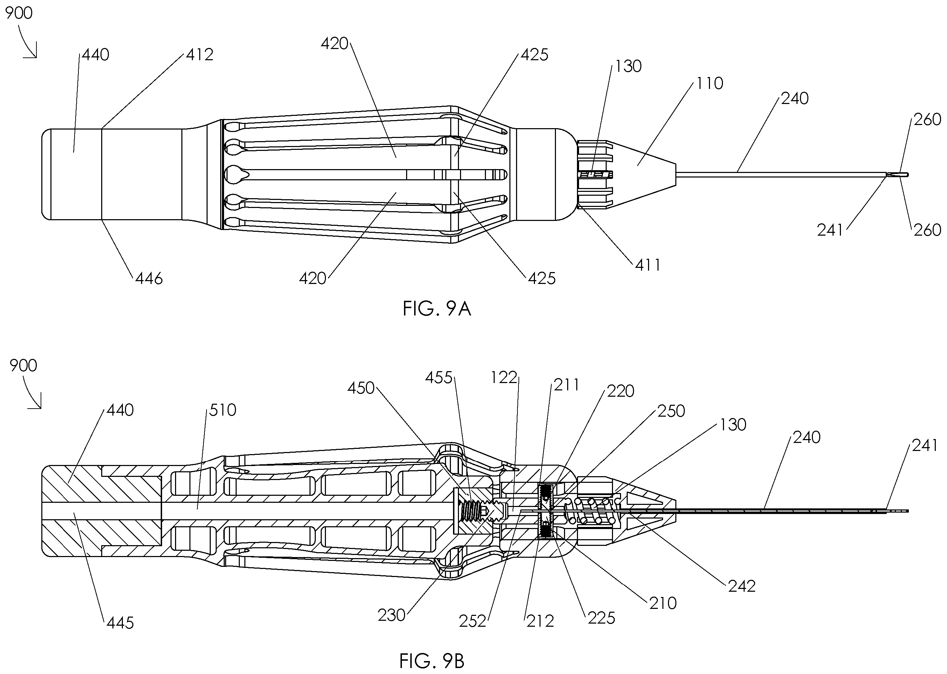

FIGs. 9A and 9B are schematic diagrams illustrating an instrument with closed jaws 900. FIG. 9A illustrates a superior view of an instrument with closed jaws 900. FIG. 9B illustrates a cross-sectional view in a sagittal plane of an instrument with closed jaws 900. Illustratively, a compression of actuation structure 410 may be configured to gradually transition assembled instrument handle 500 and assembled instrument tip 300 from an instrument with partially closed jaws 800 to an instrument with closed jaws 900. In one or more embodiments, a compression of actuation structure 410 may be configured to extend actuation structure distal end 411 relative to actuation structure proximal end 412. Illustratively, an extension of actuation structure distal end 411 relative to actuation structure proximal end 412 may be configured to extend nosecone 110 relative to handle base 105. In one or more embodiments, an extension of nosecone 110 relative to handle base 105 may be configured to extend hypodermic tube 240 relative to blank 250. Illustratively, an extension of hypodermic tube 240 relative to blank 250 may be configured to extend hypodermic tube distal end 241 over a portion of a first instrument jaw 260 and over a portion of a second instrument jaw 260. In one or more embodiments, an extension of hypodermic tube distal end 241 over a portion of a first instrument jaw 260 and over a portion of a second instrument jaw 260 may be configured to reduce a separation distance between the first instrument jaw 260 and the second instrument jaw 260 until assembled instrument handle 500 and assembled instrument tip 300 comprise an instrument with closed jaws 900. Illustratively, an extension of nosecone 110 relative to handle base 105 may be configured to expand pressure mechanism 130, e.g., an extension of nosecone 110 relative to handle base 105 may be configured to extend pressure mechanism distal end 131 relative to pressure mechanism proximal end 132. In one or more embodiments, pressure mechanism 130 may be configured to provide a force that resists an extension of nosecone 110 relative to handle base 105, e.g., pressure mechanism 130 may be configured to provide a force that facilitates a retraction of nosecone 110 relative to handle base 105.

Illustratively, a decompression of actuation structure 410 may be configured to gradually transition assembled instrument handle 500 and assembled instrument tip 300 from an instrument with closed jaws 900 to an instrument with partially closed jaws 800. In one or more embodiments, a decompression of actuation structure 410 may be configured to retract actuation structure distal end 411 relative to actuation structure proximal end 412. Illustratively, a retraction of actuation structure distal end 411 relative to actuation structure proximal end 412 may be configured to retrace nosecone 110 relative to handle base 105. In one or more embodiments, a retraction of nosecone 110 relative to handle base 105 may be configured to retract hypodermic tube 240 relative to blank 250. Illustratively, a retraction of hypodermic tube 240 relative to blank 250 may be configured to retract hypodermic tube distal end 241 off from a portion of a first instrument jaw 260 and off from a portion of a second instrument jaw 260. In one or more embodiments, a retraction of hypodermic tube distal end 241 off from a portion of a first instrument jaw 260 and off from a portion of a second instrument jaw 260 may be configured to increase a separation distance between the first instrument jaw 260 and the second instrument jaw 260 until assembled instrument handle 500 and assembled instrument tip 300 comprise an instrument with partially closed jaws 800. Illustratively, a retraction of nosecone 110 relative to handle base 105 may be configured to collapse pressure mechanism 130, e.g., a retraction of nosecone 110 relative to handle base 105 may be configured to retract pressure mechanism distal end 131 relative to pressure mechanism proximal end 132. In one or more embodiments, pressure mechanism 130 may be configured to provide a force that facilitates a retraction of nosecone 110 relative to handle base 105.

Illustratively, assembled instrument handle 500 may comprise a reusable instrument and assembled instrument tip 300 may comprise a single-use instrument. In one or more embodiments, a user may install a first assembled instrument tip 300 in assembled instrument handle 500 by inserting fixation mechanism proximal end 232 into fixation mechanism proximal housing 455. Illustratively, fixation mechanism proximal housing 455 may be configured to temporarily fix the first assembled instrument tip 300 in instrument tip housing 430, e.g., fixation mechanism proximal housing 455 may be configured to temporarily fix the first assembled instrument tip 300 in instrument tip housing 430 while the user performs a first surgical procedure. In one or more embodiments, the user may remove the first assembled instrument tip 300 from assembled instrument handle 500 by removing fixation mechanism proximal end 232 from fixation mechanism proximal housing 455. Illustratively, the user may install a second assembled instrument tip 300 in assembled instrument handle 500 by inserting fixation mechanism proximal end 232 into fixation mechanism proximal housing 455. In one or more embodiments, fixation mechanism proximal housing 455 may be configured to temporarily fix the first assembled instrument tip 300 in instrument tip housing 430, e.g., fixation mechanism proximal housing 455 may be configured to temporarily fix the first assembled instrument tip 300 in instrument tip housing 430 while the user performs a second surgical procedure.

The foregoing description has been directed to particular embodiments.

It will be apparent; however, that other variations and modifications may be made to the described embodiments, with the attainment of some or all of their advantages. Specifically, it should be noted that the principles of the present disclosure may be implemented in any system. Furthermore, while this description has been written in terms of a surgical instrument, the teachings are equally suitable to any systems where the functionality may be employed. The scope of the invention is defined by the claims.

## Claims

1. An instrument comprising:
a reusable instrument handle (500) and a replaceable single use instrument tip (300),
the reusable instrument handle (500) having
• a handle distal end (501) and a handle proximal end (502);
• an actuation structure (410) of the handle having an actuation structure distal end (411) and an actuation structure proximal end (412);
• a plurality of actuation arms (420) of the actuation structure (410);
• a fixation mechanism proximal receptacle (450) having a fixation mechanism proximal receptacle distal end (451), a fixation mechanism proximal receptacle proximal end (452), and a fixation mechanism proximal housing (455), the fixation mechanism proximal receptacle (450) disposed in the actuation structure (410);
• an instrument tip housing (430) of the actuation structure (410);
the single use instrument tip (300) having
• an instrument tip distal end and an instrument tip proximal end;
• a transitory element (100) having a transitory element distal end (101) and a transitory element proximal end (102);
• a hypodermic tube (240) having a hypodermic tube distal end (241) and a hypodermic tube proximal end (242) wherein the hypodermic tube proximal end (242) is disposed in the transitory element (100);
• a blank (250) having a blank distal end (251) and a blank proximal end (252) wherein the blank (250) is disposed in the hypodermic tube (240) and the transitory element (100); and
• a fixation mechanism (230) disposed in the transitory element (100) and the fixation mechanism proximal housing (455) of the reusable instrument handle (500); the fixation mechanism (230) having a proximal end (232),
wherein the fixation mechanism (230) of the single use instrument tip (300) and the fixation mechanism proximal receptacle (450) and the instrument tip housing (430) of the reusable instrument handle (500) are configured to removably temporarily fix the fixation mechanism (230) of the single use instrument tip (300) in the instrument tip housing (430) of the actuation structure (410) with the proximal end (232) of the fixation mechanism (230) disposed in the fixation mechanism proximal receptacle (450) of the reusable instrument handle (500).

2. The instrument of claim 1, wherein the transitory element (100) comprises an outer base (105) with an outer base aperture (108), wherein the fixation mechanism of the single use instrument tip (100) comprises a fixation mechanism distal receptable (120) providing a lock housing (125) configured to actuate within the outer base aperture (108) of the outer base (105) and wherein the blank proximal end (252) is fixed to a lock (210) disposed in the lock housing (125).

3. The instrument of claim 1 or 2, wherein the transitory element (100) comprises a nosecone (110) and wherein a portion of the hypodermic tube (240) is fixed within a portion of the nosecone (110).

4. The instrument of claim 3, wherein the nosecone (110) of the transitory element (100) has a nosecone distal end (111) and a nosecone proximal end (112); wherein the outer base (105) of the transitory element (100) has an outer base distal end (106) and an outer base proximal end (107), and wherein the outer base distal end (105) is disposed between the nosecone distal end (111) and the nosecone proximal end (112).

5. The instrument of claim 3 or 4, further comprising:
a pressure mechanism (130) of the transitory element (130) having a pressure mechanism distal end (131) and a pressure mechanism proximal end (132), wherein the pressure mechanism (130) is configured to provide a force that resists an action of the lock housing (125) within the outer base aperture (108).

6. The instrument of one of claims 3 to 5, wherein a compression of the actuation structure is configured to extend the nosecone relative to the handle base (440).

7. The instrument of claim 6, wherein the compression of the actuation structure (420) is configured to extend the hypodermic tube (240) relative to the blank (250).

8. The instrument of one of claims 3 to 5, wherein a decompression of the actuation structure is configured to retract the nosecone relative to the handle base (440).

9. The instrument of claim 8, wherein the decompression of the actuation structure (420) is configured to retract the hypodermic tube (240) relative to the blank (250).

10. The instrument of one of claims 1 to 9, wherein the transitory element (100) is manufactured from a material configured to deform if the transitory element (100) is sterilized in a medical autoclave.

11. The instrument of claim 10 wherein the material has a melting point in a range of 70° C to 100° C.

12. The instrument of claim 10 wherein the material has a melting point of less than 60°C.

13. The instrument of one of claims 8 to 12, wherein the transitory element (100) is manufactured from a material configured to retain ethylene oxide.

14. The instrument of claim 13, wherein the material has a degree of crystallinity greater than 60. 0 percent.

15. The instrument of claims 13 or 14, wherein the material is configured to retain less than 4.0 milligrams of ethylene oxide after a first sterilization by ethylene oxide and the material is configured to retain more than 4.0 milligrams of ethylene oxide after a second sterilization by ethylene oxide.

16. The instrument of one of claims 1 to 15, wherein the material of the instrument handle (500) is selected that the handle can be sterilized in a medical autoclave.

17. The instrument of one of claims 1 to 15, wherein the blank (250) comprises one or more instrument jaws (260).

18. The instrument of claim 17, wherein the instrument jaws (260) comprise forceps jaws or scissors jaws.

## Patentansprüche

1. Instrument, umfassend:
einen wiederverwendbaren Instrumentengriff (500) und eine austauschbare Einweginstrumentenspitze (300), wobei der wiederverwendbare Instrumentengriff (500) aufweist:
• ein distales Griffende (501) und ein proximales Griffende (502);
• eine Betätigungsstruktur (410) des Griffs, die ein distales Betätigungsstrukturende (411) und ein proximales Betätigungsstrukturende (412) aufweist;
• eine Vielzahl von Betätigungsarmen (420) der Betätigungsstruktur (410);
• eine proximale Fixiermechanismusaufnahme (450) mit einem distalen Ende (451) der proximalen Fixiermechanismusaufnahme, einem proximalen Ende (452) der proximalen Fixiermechanismusaufnahme und einem proximalen Fixiermechanismusgehäuse (455), wobei die proximale Fixiermechanismusaufnahme (450) in der Betätigungsstruktur (410) angeordnet ist;
• ein Instrumentenspitzengehäuse (430) der Betätigungsstruktur (410);
wobei die Einweginstrumentenspitze (300) aufweist:
• ein distales Instrumentenspitzenende und ein proximales Instrumentenspitzenende;
• ein Übergangselement (100) mit einem distalen Übergangselementende (101) und einem proximalen Übergangselementende (102);
• ein hypodermisches Röhrchen (240) mit einem distalen Ende (241) des hypodermischen Röhrchens und einem proximalen Ende (242) des hypodermischen Röhrchens, wobei das proximale Ende (242) des hypodermischen Röhrchens in dem Übergangselement (100) angeordnet ist;
• einen Rohling (250) mit einem distalen Rohlingsende (251) und einem proximalen Rohlingsende (252), wobei der Rohling (250) in dem hypodermischen Röhrchen (240) und dem Übergangselement (100) angeordnet ist; und
• einen Fixiermechanismus (230), der in dem Übergangselement (100) und dem proximalen Fixiermechanismusgehäuse (455) des wiederverwendbaren Instrumentengriffs (500) angeordnet ist; wobei der Fixiermechanismus (230) ein proximales Ende (232) aufweist,
wobei der Fixiermechanismus (230) der Einweginstrumentenspitze (300) und die proximale Fixiermechanismusaufnahme (450) und das Instrumentenspitzengehäuse (430) des wiederverwendbaren Instrumentengriffs (500) dazu ausgestaltet sind, den Fixiermechanismus (230) der Einweginstrumentenspitze (300) in dem Instrumentenspitzengehäuse (430) der Betätigungsstruktur (410) entfernbar temporär zu fixieren, wobei das proximale Ende (232) des Fixiermechanismus (230) in der proximalen Fixiermechanismusaufnahme (450) des wiederverwendbaren Instrumentengriffs (500) angeordnet ist.

2. Instrument nach Anspruch 1, wobei das Übergangslement (100) eine äußere Basis (105) mit einer äußeren Basisöffnung (108) umfasst, wobei der Fixiermechanismus der Einweginstrumentenspitze (100) eine distale Fixiermechanismusaufnahme (120) umfasst, die ein Verriegelungsgehäuse (125) bereitstellt, das ausgestaltet ist, um innerhalb der äußeren Basisöffnung (108) der äußeren Basis (105) betätigt zu werden, und wobei das proximale Rohlingsende (252) an einer Verriegelung (210) fixiert ist, die in dem Verriegelungsgehäuse (125) angeordnet ist.

3. Instrument nach Anspruch 1 oder 2, wobei das Übergangselement (100) einen Bugkonus (110) umfasst, und wobei ein Abschnitt des hypodermischen Röhrchens (240) innerhalb eines Abschnitts des Bugkonus (110) fixiert ist.

4. Instrument nach Anspruch 3, wobei der Bugkonus (110) des Übergangselements (100) ein distales Bugkonusende (111) und ein proximales Bugkonusende (112) aufweist; wobei die äußere Basis (105) des Übergangselements (100) ein distales Ende (106) der äußeren Basis und ein proximales Ende (107) der äußeren Basis aufweist, und wobei das distale Ende (105) der äußeren Basis zwischen dem distalen Bugkonusende (111) und dem proximalen Bugkonusende (112) angeordnet ist.

5. Instrument nach Anspruch 3 oder 4, ferner umfassend: einen Druckmechanismus (130) des Übergangselements (130) mit einem distalen Druckmechanismusende (131) und einem proximalen Druckmechanismusende (132), wobei der Druckmechanismus (130) dazu ausgestaltet ist, eine Kraft bereitzustellen, die einer Aktion des Verriegelungsgehäuses (125) innerhalb der äußeren Basisöffnung (108) Widerstand entgegensetzt.

6. Instrument nach einem der Ansprüche 3 bis 5, wobei eine Kompression der Betätigungsstruktur dazu ausgestaltet ist, den Bugkonus relativ zu der Griffbasis (440) zu verlängern.

7. Instrument nach Anspruch 6, wobei die Kompression der Betätigungsstruktur (420) dazu ausgestaltet ist, das hypodermische Röhrchen (240) relativ zu dem Rohling (250) zu verlängern.

8. Instrument nach einem der Ansprüche 3 bis 5, wobei eine Dekompression der Betätigungsstruktur dazu ausgestaltet ist, den Bugkonus relativ zu der Griffbasis (440) zurückzuziehen.

9. Instrument nach Anspruch 8, wobei die Dekompression der Betätigungsstruktur (420) dazu ausgestaltet ist, das hypodermische Röhrchen (240) relativ zu dem Rohling (250) zurückzuziehen.

10. Instrument nach einem der Ansprüche 1 bis 9, wobei das Übergangselement (100) aus einem Material gefertigt ist, das ausgestaltet ist, sich zu verformen, wenn das Übergangselement (100) in einem medizinischen Autoklaven sterilisiert wird.

11. Instrument nach Anspruch 10, wobei das Material einen Schmelzpunkt in einem Bereich von 70 °C bis 100 °C aufweist.

12. Instrument nach Anspruch 10, wobei das Material einen Schmelzpunkt von weniger als 60 °C aufweist.

13. Instrument nach einem der Ansprüche 8 bis 12, wobei das Übergangselement (100) aus einem Material gefertigt ist, das dazu ausgestaltet ist, Ethylenoxid zurückzuhalten.

14. Instrument nach Anspruch 13, wobei das Material einen Kristallinitätsgrad größer als 60,0 Prozent aufweist.

15. Instrument nach Anspruch 13 oder 14, wobei das Material dazu ausgestaltet ist, nach einer ersten Sterilisation durch Ethylenoxid weniger als 4,0 Milligramm Ethylenoxid zurückzuhalten, und das Material dazu ausgestaltet ist, nach einer zweiten Sterilisation durch Ethylenoxid mehr als 4,0 Milligramm Ethylenoxid zurückzuhalten.

16. Instrument nach einem der Ansprüche 1 bis 15, wobei das Material des Instrumentengriffs (500) so ausgewählt ist, dass der Griff in einem medizinischen Autoklaven sterilisiert werden kann.

17. Instrument nach einem der Ansprüche 1 bis 15, wobei der Rohling (250) eine oder mehrere Instrumentenbacken (260) umfasst.

18. Instrument nach Anspruch 17, wobei die Instrumentenbacken (260) Zangenbacken oder Scherenbacken umfassen.

## Revendications

1. Instrument comprenant :
une poignée d'instrument réutilisable (500) et un embout d'instrument à usage unique remplaçable (300), la poignée d'instrument réutilisable (500) ayant
• une extrémité distale de poignée (501) et une extrémité proximale de poignée (502) ;
• une structure d'actionnement (410) de la poignée ayant une extrémité distale de structure d'actionnement (411) et une extrémité proximale de structure d'actionnement (412) ;
• une pluralité de bras d'actionnement (420) de la structure d'actionnement (410) ;
• un logement proximal de mécanisme de fixation (450) ayant une extrémité distale de logement proximal de mécanisme de fixation (451), une extrémité proximale de logement proximal de mécanisme de fixation (452), et un boîtier proximal de mécanisme de fixation (455), le logement proximal de mécanisme de fixation (450) étant disposé dans la structure d'actionnement (410) ;
• un boîtier d'embout d'instrument (430) de la structure d'actionnement (410) ;
l'embout d'instrument à usage unique (300) ayant
• une extrémité distale d'embout d'instrument et une extrémité proximale d'embout d'instrument ;
• un élément transitoire (100) ayant une extrémité distale d'élément transitoire (101) et une extrémité proximale d'élément transitoire (102) ;
• un tube hypodermique (240) ayant une extrémité distale de tube hypodermique (241) et une extrémité proximale de tube hypodermique (242) l'extrémité proximale de tube hypodermique (242) étant disposée dans l'élément transitoire (100) ;
• une ébauche (250) ayant une extrémité distale d'ébauche (251) et une extrémité proximale d'ébauche (252), l'ébauche (250) étant disposée dans le tube hypodermique (240) et l'élément transitoire (100) ; et
• un mécanisme de fixation (230) disposé dans l'élément transitoire (100) et le boîtier proximal de mécanisme de fixation (455) de la poignée d'instrument réutilisable (500) ; le mécanisme de fixation (230) ayant une extrémité proximale (232),
le mécanisme de fixation (230) de l'embout d'instrument à usage unique (300) et le logement proximal de mécanisme de fixation (450) et le boîtier d'embout d'instrument (430) de la poignée d'instrument réutilisable (500) étant conçus pour fixer de manière amovible temporaire le mécanisme de fixation (230) de l'embout d'instrument à usage unique (300) dans le boîtier d'embout d'instrument (430) de la structure d'actionnement (410) avec l'extrémité proximale (232) du mécanisme de fixation (230) disposée dans le logement proximal de mécanisme de fixation (450) de la poignée d'instrument réutilisable (500).

2. Instrument selon la revendication 1, l'élément transitoire (100) comprenant une base externe (105) avec une ouverture de base externe (108), le mécanisme de fixation de l'embout d'instrument à usage unique (100) comprenant un logement distal de mécanisme de fixation (120) fournissant un boîtier de verrouillage (125) conçu pour s'actionner dans l'ouverture de base externe (108) de la base externe (105) et l'extrémité proximale d'ébauche (252) étant fixée à un verrou (210) disposé dans le boîtier de verrouillage (125).

3. Instrument selon la revendication 1 ou 2, l'élément transitoire (100) comprenant un cône avant (110) et une partie du tube hypodermique (240) étant fixée dans une partie du cône avant (110).

4. Instrument selon la revendication 3, le cône avant (110) de l'élément transitoire (100) ayant une extrémité distale de cône avant (111) et une extrémité proximale de cône avant (112) ; la base externe (105) de l'élément transitoire (100) ayant une extrémité distale de base externe (106) et une extrémité proximale de base externe (107), et l'extrémité distale de base externe (105) étant disposée entre l'extrémité distale de cône avant (111) et l'extrémité proximale de cône avant (112).

5. Instrument selon la revendication 3 ou 4, comprenant en outre :
un mécanisme de pression (130) de l'élément transitoire (130) ayant une extrémité distale de mécanisme de pression (131) et une extrémité proximale de mécanisme de pression (132), le mécanisme de pression (130) étant conçu pour fournir une force qui résiste à une action du boîtier de verrouillage (125) dans l'ouverture de base externe (108).

6. Instrument selon l'une des revendications 3 à 5, une compression de la structure d'actionnement étant conçue pour étendre le cône avant par rapport à la base de poignée (440).

7. Instrument selon la revendication 6, la compression de la structure d'actionnement (420) étant conçue pour étendre le tube hypodermique (240) par rapport à l'ébauche (250).

8. Instrument selon l'une des revendications 3 à 5, une décompression de la structure d'actionnement étant conçue pour rétracter le cône avant par rapport à la base de poignée (440).

9. Instrument selon la revendication 8, la décompression de la structure d'actionnement (420) étant conçue pour rétracter le tube hypodermique (240) par rapport à l'ébauche (250).

10. Instrument selon l'une des revendications 1 à 9, l'élément transitoire (100) étant fabriqué à partir d'un matériau conçu pour se déformer si l'élément transitoire (100) est stérilisé dans un autoclave médical.

11. Instrument selon la revendication 10, le matériau ayant un point de fusion dans une plage de 70 °C à 100 °C.

12. Instrument selon la revendication 10, le matériau ayant un point de fusion de moins de 60 °C.

13. Instrument selon l'une des revendications 8 à 12, l'élément transitoire (100) étant fabriqué à partir d'un matériau conçu pour retenir l'oxyde d'éthylène.

14. Instrument selon la revendication 13, le matériau ayant un degré de cristallinité supérieur à 60,0 pour cent.

15. Instrument selon la revendication 13 ou 14, le matériau étant conçu pour retenir moins de 4,0 milligrammes d'oxyde d'éthylène après une première stérilisation par oxyde d'éthylène et le matériau étant conçu pour retenir plus de 4,0 milligrammes d'oxyde d'éthylène après une seconde stérilisation par oxyde d'éthylène.

16. Instrument selon l'une des revendications 1 à 15, le matériau de la poignée d'instrument (500) étant sélectionné de sorte que la poignée puisse être stérilisée dans un autoclave médical.

17. Instrument selon l'une des revendications 1 à 15, l'ébauche (250) comprenant une ou plusieurs mâchoires d'instrument (260).

18. Instrument selon la revendication 17, les mâchoires d'instrument (260) comprenant des mâchoires de forceps ou des mâchoires de ciseaux.
